# EUROPEAN PATENT APPLICATION

(11) **EP 2 457 990 A1**
(43) Date of publication of application: **30.05.2012**
(21) Application number: 10192909.9
(22) Date of filing: 29.11.2010
(51) Int. Cl.: C12M 1/08

(54) **Process and device for the production of gluconic acid**

(71) Applicant: Sika Technology AG, 6340 Baar (CH)
(72) Inventor: The designation of the inventor has not yet been filed
(74) Representative: Sika Patent Attorneys

(57) **Abstract**

Subject of the invention is a process for the enzymatic oxidation of a substrate. Specifically, the invention relates to the production of gluconate, wherein the gluconate is produced in an enzymatic reaction from glucose, wherein the reaction is carried out in a jet loop reactor comprising at least one static mixer and at least one ejector for enriching the reaction mixture with oxygen. The invention also relates to a jet loop reactor comprising at least one static mixer and at least one ejector for enriching the reaction mixture with oxygen.

## Description

The invention relates to enzymatic oxidation processes and devices for carrying out the processes. Specifically, the invention relates to a process for the production of gluconate from glucose in an enzymatic reaction and to devices for carrying out the reaction.

### State of the art

Gluconic acid has the formula HOCH₂-(CHOH)₄-COOH and is one of the 16 stereoisomers of 2,3,4,5,6-pentahydroxyhexanoic acid. The salts of gluconic acid are known as "gluconates". Gluconic acid, gluconate salts, and gluconate esters occur widely in nature because they arise from the oxidation of glucose. Gluconic acid is a mild acid with many technical applications, which comprise its use as a complexing agent in industrial cleaning of metal surfaces, in the textile industry, in detergents and in concrete, as a food additive in beverages and also in bread and feed, and in pharmaceutical preparations.

The production of gluconic acid in an industrial scale is carried out using a fermentation process in the presence of microorganisms, such as *Aspergillus* or *Gluconobacter* species. In these fungi or bacteria respectively, glucose is converted into gluconic acid by an enzymatic complex consisting of glucose oxidase and catalase.

Since fermentation reactions using fungi or bacteria are relatively complicated, processes have been developed in which the reaction is catalysed *in vitro* by the enzymes. WO 96/35800 suggests a process for the enzymatic conversion of glucose into gluconic acid, in which a 10% (w/v) glucose solution is converted into gluconic acid. The conversion takes place in the presence of glucose oxidase and catalase using an excess of activity units of catalase relative to oxidase. When using isolated enzymes, the turnover is not very high and the yield of gluconate is relatively low. Thus various modifications of the process have been suggested to improve the yield. For example, FR-A-2177931 and EP-A-0017708 suggest the use of immobilized enzymes. FR-A-2029645 suggests combining the reaction with separation of the product by electrodialysis.

Even with the proposed modifications, the yield and turnover are still not satisfactory. In view of the technical applications of gluconate, it would be highly desirable to provide methods in which the yield and turnover are improved.

### Problem underlying the invention

The problem underlying the invention is to provide a process and a device for the production of an oxidized substrate, such as gluconate, which overcome the above-mentioned problems. The invention shall provide an efficient process for producing the oxidized substrate, such as gluconate, in an enzymatic reaction at a high yield. The enzymatic turnover shall be high and the process shall require a low reaction time. The process shall be applicable for industrial scale production of gluconate. Preferably, the process shall also be advantageous regarding consumption of energy and consumable materials, such as reactants and enzymes.

Another problem underlying the invention is to provide a relatively simple process, which avoids multiple treatment steps. Specifically, time-consuming and laborious purification steps, such as an anion exchange chromatography step, shall be avoided.

### Disclosure of the invention

Surprisingly, it was found that the problem underlying the invention is overcome by processes, jet loop reactors and uses according to the claims. Further embodiments of the invention are outlined throughout the description.

Subject of the invention is a process for oxidizing a substrate, wherein the oxidized substrate is produced in an enzymatic reaction from the substrate, wherein the reaction is carried out in a jet loop reactor, the reactor comprising at least one static mixer and at least one ejector for enriching the reaction mixture with oxygen.

In a preferred embodiment, the process is a process for the production of gluconate, wherein the gluconate is produced in an enzymatic reaction from glucose, wherein the reaction is carried out in a jet loop reactor comprising at least one static mixer and at least one ejector for enriching the reaction mixture with oxygen.

Since gluconic acid is a mild acid, it is usually in equilibrium with gluconate at the reaction conditions. Thus, the distinction between gluconic acid and gluconate under the reaction conditions is generally not reasonable. As used herein, the terms gluconic acid and gluconate are used interchangeably. Thus, when it is referred to "gluconic acid" or "gluconate" herein, reference is made to the total of gluconic acid and gluconate.

The reactor comprises an aqueous reaction mixture. The reaction mixture comprises the dissolved or dispersed reactants and intermediates, such as glucose, oxygen, hydrogen peroxide, and the product gluconate. Enzymes are dissolved in the liquid reaction mixture, if they are not immobilized. Further additives might be present for supporting or monitoring the process, such as salts, coenzymes, enzyme stabilizers, pH buffers, pH indicators and the like.

The inventive process is an enzymatic process. The enzymes may be provided in isolated form or as crude preparations, or mixtures thereof. The enzymatic process may also use cell extracts or fractions thereof. However, the process is not an *in vivo* process using viable cells.

In a preferred embodiment of the invention, the enzymes catalyzing the reaction are glucose oxidase and catalase. Glucose oxidase catalyzes the oxidation of β-D-glucose to D-glucono-ω-lactone and hydrogen peroxide. It also catalyzes the subsequent conversion of D-glucono-ω-lactone into gluconic acid. Catalase decomposes the hydrogen peroxide formed in the first reaction.

Preferably, at least one of these enzymes, preferably both, is a fungal enzyme. Preferably, the fungus is *Aspergillus* or *Penicillium.* More preferably, the enzymes are glucose oxidase and catalase from *Aspergillus niger. A. niger* glucose oxidase (CAS 9001-37-0) is a homodimer with a molecular weight of about 150 kDa containing prosthetic groups of FAD. *A. niger* catalase (CAS 9001-05-2) is a tetramer having a molecular weight of about 340 kDa. Both enzymes are commercially available, for example from Genencor, DK or Merck Chemicals Ltd., UK.

The enzymes to be used for the conversion of glucose into gluconic acid can be derived from a single strain or can be separately derived from different strains and subsequently mixed to obtain the desired mixture of the enzymes. The enzymes may be recombinant proteins or may be isolated from fungi or bacteria, respectively. The fungal strains used for the production of the enzyme preparations of the invention may be wild type strains or strains modified by classical mutagenesis and/or by the use recombinant DNA technology. The modifications may include modification of the properties of the enzymes through chemical modification, such as glycosylation.

The enzymes may be derivatives and/or mutants of wild-type enzymes. The terms "derivatives" and "mutants" refer to enzymes, which are different from wild-type enzymes due to amino acid substitutions, deletions, insertions or modifications. The derivatives and/or mutants have glucose oxidase or catalase activity. Preferably, the activity is more than 50%, more preferably more than 100% or more than 150% of the activity of the corresponding wild-type enzymes. The derivatives and mutants can be obtained by recombinant cloning techniques or by chemical modification.

The enzyme concentration in the solution is adjusted to obtain an optimized yield. For example, it may be selected between 0.05 and 2% (w/w). Preferably, the concentration of the glucose oxidase in the reaction mixture is 0.1 % to 1.5 (w/w), preferably between 0.2 and 1% (w/w). Preferably, the concentration of the catalase in the reaction mixture is 0.05% to 1.5% (w/w), preferably between 0.2 and 1% (w/w).

Preferably, the enzymes catalase and glucose oxidase are present in an activity ratio in excess of 200 IU/SU, respectively, wherein IU is defined as the International catalase Unit and SU is defined as the Sarett glucose oxidase unit. More preferably, the activity ratio of the enzyme preparation of the invention is higher than 400 IU/SU. Even more preferable is an activity ratio in excess of 700 IU/SU.

The enzymes can be used in soluble form and/or can be immobilized. However, in a preferred embodiment the enzymes are not bound to a carrier, such that they are not immobilized. The enzymes thus pass the loop and the ejector and are thoroughly mixed with the oxygen and reactant. The immobilization of enzymes may be advantageous when the enzymes are relatively instable and may deteriorate when passing the ejector.

In a preferred embodiment, the pH of the reaction mixture is between 4.5 and 6, more preferably between 4.8 and 5.8, most preferably between 5.0 and 5.5.

In a preferred embodiment of the invention, the reaction is carried out at a temperature between 30°C and 100°C, more preferably between 40°C and 80°C or between 40°C and 60°C. The temperature is preferably adjusted such that an optimal turnover of glucose to gluconate is obtained and depends upon the adjustment of pH. At preferred pH of 5.0-5.5 temperature optimum is in between 40°C to 60°C.

In a preferred embodiment of the invention, the air over pressure in the reaction chamber is between 0.05 bar and 2 bar, more preferably between 0.1 bar and 1.5 bar or between 0.5 and 1.2 bar.

In a preferred embodiment of the invention, the oxygen level in the reaction mixture is above 30 mg/l. More preferably, the oxygen level is above 40 mg/l or above 50 mg/l. The oxygen level is adjusted through the injector.

In a preferred embodiment of the invention, the glucose concentration in the reaction mixture is between 10% and 80% (w/w), more preferably between 30% and 70% or between 50% and 65% (w/w). The glucose can be provided in the form of glucose syrup (corn syrup). Glucose syrup is commercially available, for example from CornProducts International, US. Glucose syrup mainly consists of glucose, but may comprise minor levels of fructose. Preferably, the corn syrup comprises about 100% glucose, or at least more than 95% glucose.

Preferably, the sugar comprised in the reaction mixture is essentially glucose. Preferably, the percentage of glucose in the reaction mixture is more than 90%, more than 95% or more than 98% (w/w), based on the total amount of sugars dissolved. Thus the inventive conversion of glucose to gluconate is the main reaction in the reaction mixture.

In a specific embodiment of the invention, the reaction mixture comprises 10 to 80% (w/w) glucose, 0.1 % to 1.5 (w/w) glucose oxidase and 0.05% to 1.5% (w/w) catalase.

Another subject of the invention is a jet loop reactor comprising at least one static mixer and at least one ejector for enriching the reaction mixture with oxygen.

According to the invention, it was found that the turnover of glucose and yield of gluconate can be greatly increased, when the enzymatic reaction is carried out in a jet loop reactor comprising at least one static mixture and at least one ejector. It was found that in such a device the oxygen level in the reaction mixture can be increased significantly. As a result, it was found that the overall reaction is strongly accelerated.

The reactor comprises a reaction chamber, such as the interior of a vessel, and additional components and devices attached to the reactor chamber.

A static mixer is a device for mixing and/or homogenizing a fluid material. The device consists of mixer elements contained in a cylindrical tube or squared housing. As the fluid stream moves through the mixer, the mixer elements blend the materials dissolved or dispersed in the fluid stream. Static mixer elements consist of a series of baffles that are made from metal or plastics. The mixer housing can be made from glass, metal or plastic. In the inventive reactor, the reaction mixture from the reaction chamber is led through the static mixer. The inlet is positioned in the reaction chamber and in the liquid phase.

An ejector (jet stream device) comprises a converging-diverging nozzle. A motive fluid is introduced through an injector. The nozzle converts the pressure energy of a motive fluid to velocity energy, which creates a low pressure zone that draws in and entrains a suction fluid. After passing through the throat of the injector, the mixed fluid expands and the velocity is reduced which results in recompressing the mixed fluids by converting velocity energy back into pressure energy. According to the invention, the motive fluent is the reaction mixture and the suction fluid is gaseous and comprises oxygen. In the ejector, the reaction mixture is enriched with oxygen.

The inventive jet loop reactor comprises at least one loop (or loop arrangement) through which the reaction mixture circulates. Thereby, the reaction mixture is continuously withdrawn from the reaction chamber and subsequently reintroduced into the reaction chamber. Usually, the loop comprises at least one component or unit in which the reaction mixture is physically or chemically affected. The loop also comprises appropriate connecting means, such as pipes, hoses or tubes. The liquid flow may be supported by appropriate means, such as a pump.

The inventive device comprises an ejector unit, through which a portion of the reaction mixture is continuously removed from the reaction chamber, passes the ejector and is reintroduced into the reaction chamber. Thus the ejector is part of the loop. It is positioned at the end of the loop (in flow direction). In principle, jet loop reactors are known in the art and described for example in US 2004/0013595 A1 or Moresi et al., "The Ejector Loop Fermenter: Description and Performance of the Apparatus", Biotechnology and Bioengineering, 1983, p2889-2904.

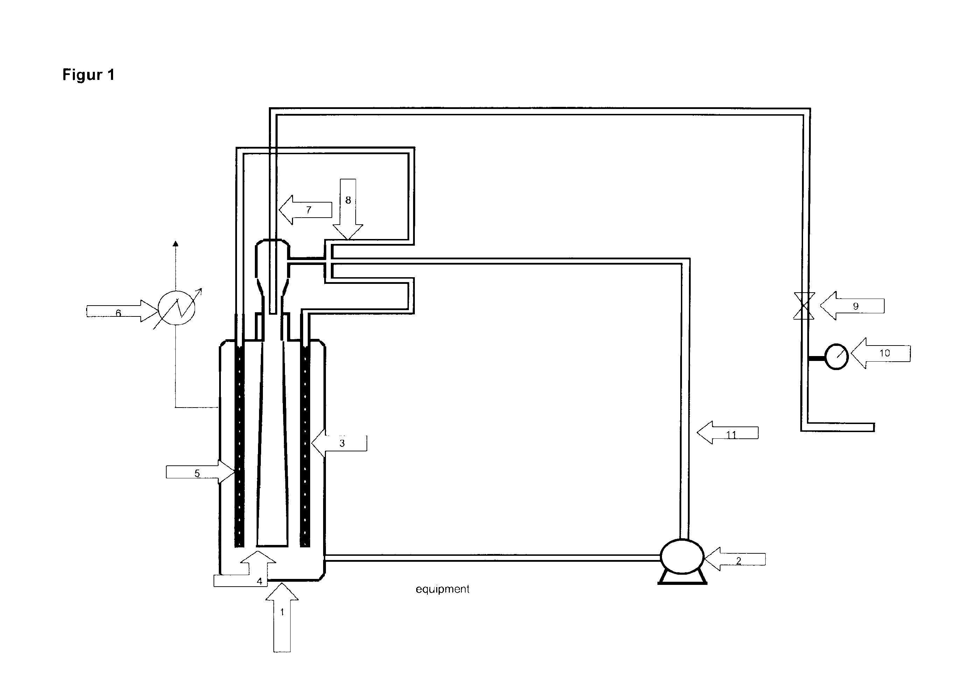

According to the invention, it is preferred that the ejector ejects the gas/liquid mixture directly into the reaction chamber. An alternative arrangement, in which the ejector is not directly inserted into the reaction chamber, is also conceivable (as shown in Fig. 1 of Moresi et al.). However, the prior art does not disclose jet loop reactors in combination with static mixers as in the present invention.

In a preferred embodiment of the invention, at least one loop between the reaction chamber and the ejector encompasses at least one static mixer. Preferably, the reactor comprises at least one loop arrangement for continuously withdrawing reaction mixture from the reaction chamber, subsequently passing the reaction mixture through at least one static mixture, and subsequently reintroducing the reaction mixture into the reaction chamber through the at least one ejector. This embodiment is advantageous, because the intermingling forces of the static mixer and the ejector are combined in one loop. Thus provide an ejected mixture is obtained, which is thoroughly admixed and homogenized and comprises a high oxygen concentration.

In the inventive jet loop reactor, the at least one loop may not encompass a static mixer. This means, when passing the at least one loop, the reaction mixture does not pass the static mixer. Preferably, the inlet and outlet of the static mixer are then positioned within the reaction chamber Such a loop connection may be advantageous in order to supply large amounts of reaction mixture to the ejector. The ejector enriches the reaction mixture with oxygen and a static mixer, which is not part of this loop (but may be part of another loop) thoroughly mixes the liquid in the reaction chamber.

In a preferred embodiment, the oxygen, which is fed into the ejector as the suction fluid, is provided as part of a gas comprising oxygen, preferably air. However, the gaseous mixture may also comprise a higher level of oxygen than in air or even pure oxygen.

In a preferred embodiment of the invention, the inventive reactor comprises at least two static mixers and/or ejectors. The reactor may also comprise 3, 4, 5, or even more static mixers and/or ejectors. The reactor may also comprise at least two loops, or 3, 4, 5 or more loops. The reactor may comprise at least one loop passing a static mixer and at least one loop not passing a static mixer.

In a preferred embodiment of the invention, the reactor comprises two loops passing static mixers and one loop, which does not pass a static mixer.

When the device comprises two loops or more, the reaction mixture streams are preferably combined before being injected into the ejector. Preferably, the streams of reaction mixture are combined in a flow divider/combiner valve or other known means for combining liquid streams. In another embodiment, each loop is connected to a corresponding ejector unit, such that it is not necessary to combine streams from different loops.

In the inventive device, the reaction mixture is enriched with oxygen in the ejector and thoroughly mixed in the static mixer, especially when both components are combined in one loop. The oxygen level in the reaction mixture can be increased significantly, for example to levels of about 50 mg/l. It is believed that the overall interfacial area between the reactants, the enzymes and oxygen in the reaction mixture is increased, which results in an improvement of the reaction kinetics. It was found that the velocity of the reaction can thereby be increased to about three times of the velocity of a reaction carried out with conventional stirring equipment. Thus the inventive reaction requires significantly less time, which is highly advantageous for industrial scale production and also regarding energy consumption. The capacity of a conventional reactor is increased and the waste of materials, such as water and enzymes, which deteriorate depending on the time used, is reduced.

The inventive reactor may comprise additional components, such as coolers, heaters and monitoring equipment for regulating the temperature in the reaction chamber, pipes, hoses and tubes for joining the components, a device for supplying oxygen and reactants, valves for adjusting the flow of the components, such as the reaction mixture and gases, and inlets and outlets for supplying reactants into the reactor. The overall flow may be regulated through a pump or other known means.

When carrying out the process with immobilized enzymes, they are not part of the mobile reaction mixture and are not fed through the loops and into the ejector. In this embodiment, there is no risk that enzyme precipitate and form deposits in the pipes, tubes and ejector. Further, a decrease of enzyme activity due to damage in the ejector is avoided.

Preferably, the reaction is a batch reaction. This means that after combining the reactants in the reactor, the reaction proceeds until a major portion of the glucose is converted into gluconate. The progression of the reaction may be monitored, for example by determining the acid level or pH value. After termination of the reaction, the gluconate can be isolated by known methods, such as precipitation and crystallization. The product may be subjected to further purification, for example by washing or recrystallization.

Another subject of the invention is the use of an inventive jet stream reactor for the production of gluconate from glucose.

The inventive enzymatic oxidation is described herein in detail for conversion of glucose to gluconic acid. However, the novel inventive jet loop reactor can be applied for any enzymatic oxidation reaction. In principle, the reaction conditions can be adjusted as outlined above for the gluconic acid synthesis. The inventive reactor also promotes enzymatic oxidation reactions, because as shown above, it provides the reaction mixture with a high oxygen level and is believed to increase the overall contact area of the reactants. Substrates for oxidation may be alcohols, specifically carbohydrates. The substrates may be low molecular weight organic compounds, which are soluble in aqueous solution. The reaction is carried out in the presence of at least one oxidase.

Exemplified embodiments of the invention and aspects of the invention are shown in the figures.
Figure 1 shows schematically, and in exemplified form an inventive reactor for carrying out the inventive reaction. The reaction chamber (1) comprises two static mixers (3, 5). At the top of the reaction chamber, an ejector (4) is positioned with an opening reaching into the reaction chamber. The arrangement of the static mixers and the ejector is such that the inlets and outlets are positioned at the lower portion of the reaction chamber, such that finely distributed oxygen can move upwards through the reaction chamber, and such that reactants, which might accumulate at the bottom of the reaction chamber, are thoroughly homogenized. The temperature of the reaction chamber is adjusted by a thermostat, cooler or heater (6). The ejector comprises a supply pipe (7) for supplying the suction fluid, which comprises oxygen, into the ejector. The oxygen supply is regulated through regulator (10) and valve (9). In the embodiment shown in Fig. 1, an additional loop (11) is present supplies additional reaction mixture from the reaction chamber to the ejector without passing a static mixer. The flow is supported by pump (2). The supply streams of reaction mixture are joined in a flow split/divisor/combiner (8).

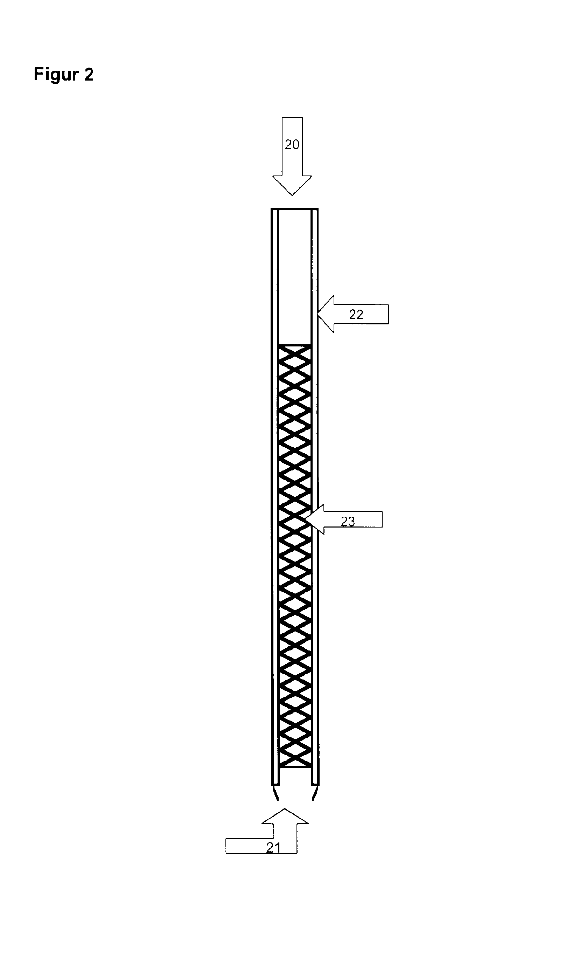

Figure 2 shows schematically the static mixer (3, 5) for use in the inventive reactor. The static mixer comprises an inlet (21) and an outlet (20), a glass cylinder (22) and mixer elements (23) positioned in the glass cylinder. Due to the movement of mixer elements (23), the reaction mixture is led upwards to the outlet of the mixer.

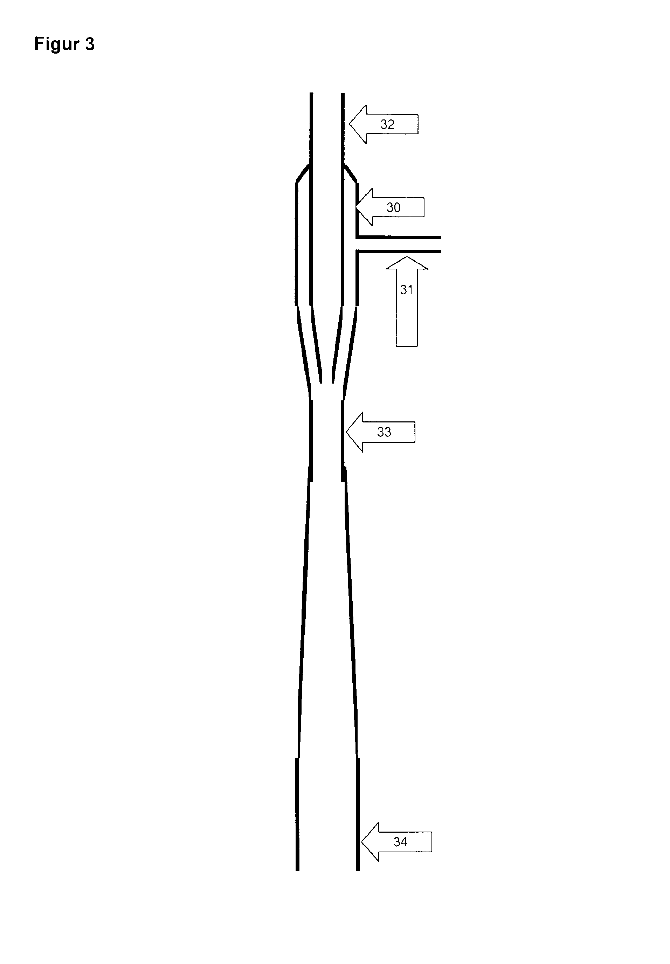

Figure 3 shows a schematic view of the ejector useful in the inventive device. The motive fluid, which is the reaction mixture, enters the ejector through a liquid feed opening (31). The oxygen, which is the suction liquid, is supplied through gas feed inlet (32). Both fluids mix in a mixing chamber (30) and enter constriction area (33). After passing constriction area (33), the mixture enters expansion section (34), from which it is reintroduced into the reaction chamber. In the ejected reaction mixture, the contact area between the reactants and oxygen is increased, such that upon contact with the enzymes, the reaction is accelerated.

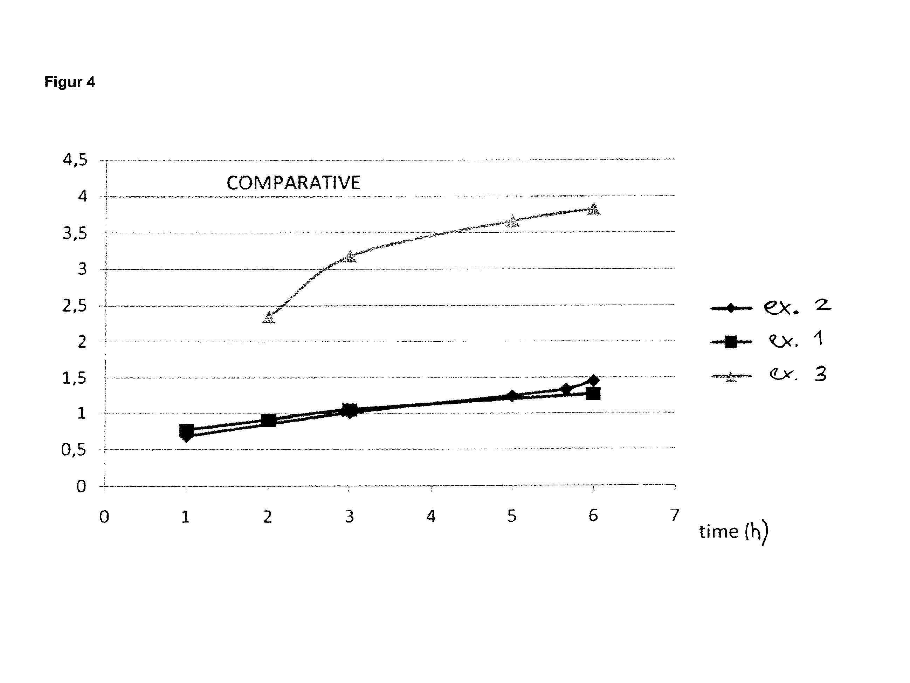

Figure 4 shows a graphic view of the amount of gluconic acid (in mg KOH/g content) obtained versus time (in hours) according to examples 1, 2 and 3. The upper line (triangles) relates to inventive experiment 3. The lower lines relate to experiment 1 (squares) and 2 (rhombus).

### Examples

### Comparative Example 1: Batch Stirred Tank Reactor (STR)

1000 g of glucose syrup from Corn Products (1130) at 58% (w/w) of D-glucose content has been added to a batch STR reactor (self constructed) equipped with thermometer and pH electrode. The solution was heated to 45°C upon stirring at 200 rpm. After reaching the desired temperature, 7 g of glucose oxidase (GO 1500L, Genencor) and 7 g catalase (CA 1000L, Genencor) were added to the stirred solution, which was set to time zero. The reaction chamber was pressurised with compressed air, using a 20 L dental compressor from Schulz at 0.4 bar over pressure. Oxidation was monitored by immediate pH decrease, which was neutralized with 0.1 N KOH (Merck KG) when inferior to pH 5.1 up to a pH of 5.5. Samples were taken at 1, 3, 5, 5 h 45' and 6 hours and KOH consumption noted (1, 1.5, 1.8, 2 and 2.1 mL, respectively). The results are shown in table 1 below.

**Table 1 Production of gluconic acid with conventional reactor according to example 1:**

| **t [h]** | **Amount Product** | **Equivalents** | **Vol. KOH** |
|---|---|---|---|
| | **[mg KOH/g content]** | **[g]** | **[ml]** |
| 1 | 0,771 | 0,0154 | 1,1 |
| 2 | 0,911 | 0,0183 | 1,3 |
| 3 | 1,052 | 0,0211 | 1,5 |
| 5 | 1.219 | 0,0247 | 1.7 |
| 6 | 1,261 | 0,0253 | 1,8 |

### Comparative Example 2: Static Pump STR Reactor

1000 g of glucose syrup from Corn Products (1130) at 58% (w/w) of D-glucose content has been added to a batch static pump reactor (self constructed) comprising a static mixer and equipped with thermometer and pH electrode. The solution was heated to 45°C after which 7 g of glucose oxidase (GO 1500L, Genencor) and 7 g catalase (CA 1000L, Genencor) were added mixed solution, which was set to time zero. The reaction chamber was pressurised with compressed air, using a 20 L dental compressor from Schulz at 0.4 bar over pressure. Oxidation was monitored by immediate pH decrease, which was neutralized with 0.1 N KOH (Merck KG) when inferior to pH 5.1 up to a pH of 5.5. Samples were taken at 1, 2, 3, 5, and 6 hours and KOH consumption noted (1.1, 1.3, 1.5, 1.7 and 1.8mL). The results are shown in table 2 below.

**Table 2 Production of gluconic acid with mixed stirrer reactor according to example 2:**

| **t [h]** | **Amount Product** | **Equivalents** | **Vol. KOH** |
|---|---|---|---|
| | **[mg KOH/g content]** | **[g]** | **[ml]** |
| 1 | 0,691 | 0,0142 | 1 |
| 3 | 1,012 | 0,0211 | 1,5 |
| 5 | 1,244 | 0,025 | 1,8 |
| 5,66 | 1,333 | 0,028 | 2 |
| 6 | 1,452 | 0,029 | 2,1 |

### Inventive Example 3: Static Pump Jet Ejector Reactor (SPE)

1000 g of glucose syrup from Corn products (1130) at 58% (w/w) of D-glucose content has been added to the new static pump jet ejector reactor of the invention (self constructed), equipped with thermometer and pH electrode. The solution was heated to 45°C after which 7 g of glucose oxidase (GO 1500L, Genencor) and 7 g catalase (CA 1000L, Genencor) were added mixed solution, which was set to time zero. The reaction chamber was pressurised with compressed air, using a 20 L dental compressor from Schulz at 0.4 bar over pressure. Oxidation was monitored by immediate pH decrease, which was neutralized with 0.1 N KOH when inferior to pH 5.1 up to a pH of 5.5. Samples were taken at 2, 3, 5, and 6 hours and KOH consumption noted (3.3, 4.5, 5.2 and 5.4 mL). The results are shown in table 3 below.

**Table 3: Production of gluconic acid with inventive reactor according to example 3:**

| **t [h]** | **Amount Product** | **Equivalents** | **Vol. KOH** |
|---|---|---|---|
| | **[mg KOH/g content]** | **[g]** | **[ml]** |
| 2 | 2,35 | 0,047 | 3,3 |
| 3 | 3,18 | 0,064 | 4,5 |
| 5 | 3,66 | 0,074 | 5,2 |
| 6 | 3,82 | 0,072 | 5,4 |

The results of examples 1, 2 and 3 are summarized in table 4 below. Table 1 also includes the oxygen levels determined in the reaction mixtures. A corresponding graphic view of the amounts of gluconic acid is provided in figure 4. The experiments show, that the oxidation reaction is much faster with the inventive reactor of example 3 and the yield is significantly higher. An increased oxygen level is observed in inventive example 3.

**Table 4: Summary of results of examples 1, 2 and 3: Amounts of gluconic acid produced at various time points as determined by titration (in mg KOH/g content) and oxygen levels as determined in reaction mixtures:**

| **t [h]** | **1** | **3** | **5** | **6** | **oxygen dissolved [mg/L]** |
|---|---|---|---|---|---|
| Example 3 inventive | 2.05 | 3.18 | 3.66 | 3.82 | 50 |
| Example 2 comparative | 0.69 | 1.01 | 1.24 | 1.45 | 20 |
| Example 1 comparative | 0.77 | 1.05 | 1.21 | 1.26 | 2.6 |

## Claims

1. A process for oxidizing a substrate, wherein the oxidized substrate is produced in an enzymatic reaction from the substrate, wherein the reaction is carried out in a jet loop reactor, the reactor comprising at least one static mixer and at least one ejector for enriching the reaction mixture with oxygen.

2. The process according to claim 1, wherein gluconate is produced in an enzymatic reaction from glucose.

3. The process according to at least one of the preceding claims, wherein the enzymes catalyzing the reaction are glucose oxidase and catalase.

4. The process of claim 3, wherein the enzymes are A. niger glucose oxidase and catalase or derivatives and/or mutants thereof.

5. The process according to at least one of the preceding claims, wherein the enzymes are bound to a carrier.

6. The process according to at least one of the preceding claims, wherein the reaction is carried out at a temperature between 25°C and 80°C.

7. The process according to at least one of the preceding claims, wherein the air over pressure in the device is between 0.05 and 2 bar.

8. The process according to at least one of the preceding claims, wherein the oxygen level in the reaction mixture is above 30 mg/l.

9. The process according to at least one of the preceding claims, wherein the glucose concentration in the reaction mixture is between 10 and 70 % (w/w).

10. A jet loop reactor, comprising at least one static mixer and at least one ejector for enriching the reaction mixture with oxygen.

11. The jet loop reactor claim 10, comprising at least one loop arrangement for continuously withdrawing reaction mixture from the reaction chamber, subsequently passing the reaction mixture through the at least one static mixture, and subsequently reintroducing the reaction mixture into the reaction chamber through the at least one ejector.

12. The jet loop reactor of claim 10 and/or 11, wherein the static mixer is positioned in the reaction chamber.

13. The jet loop reactor according to at least one of claims 10 to 12, comprising at least one loop arrangement, through which the reaction mixture is transferred to the ejector without passing a static mixer.

14. Use of a jet loop reactor according to at least one of claims 10 to 13 for the production of gluconate from glucose.
